# EUROPEAN PATENT APPLICATION

(11) **EP 1 063 288 A2**
(43) Date of publication of application: **27.12.2000**
(21) Application number: 00112618.4
(22) Date of filing: 14.06.2000
(51) Int. Cl.: C12N 1/20, C12P 13/08

(54) **Method for producing L-lysine**

(30) Priority: 15.06.1999 JP 16837699
(71) Applicant: Ajinomoto Co., Inc., Tokyo 104 (JP)
(72) Inventor: Tsujimoto, Nobuharu, c/o Ajinomoto Co., Inc., Kawasaki-shi, Kanagawa (JP); Yasueda, Hisashi, c/o Ajinomoto Co., Inc., Kawasaki-shi, Kanagawa (JP); Kawahara, Yoshio, c/o Ajinomoto Co., Inc., Kawasaki-shi, Kanagawa (JP); Sugimoto, Shinichi, c/o Ajinomoto Co., Inc., Kawasaki-shi, Kanagawa (JP)
(74) Representative: Strehl Schübel-Hopf & Partner

(57) **Abstract**

A method for producing L-lysine, which comprises culturing a methanol-assimilating thermotolerant *Bacillus* bacterium which has L-lysine-producing ability and is vitamin B₁₂ non-auxotrophic in a medium to produce and accumulate L-lysine in a culture and collecting the L-lysine from the culture.

## Description

### BACKGROUND OF THE INVENTION

The present invention relates to a method for producing L-lysine. In particular, it relates to a method for producing L-lysine utilizing a methanol-assimilating thermotolerant *Bacillus* bacterium which is vitamin B₁₂ non-auxotrophic. L-Lysine is widely used as a feed additive and so forth.

The current main stream of the L-lysine production is the fermentative production utilizing L-lysine-producing bacteria belonging to the genus *Brevibacterium, Corynebacterium, Bacillus* or *Escherichia.* In these methods, the temperature of the medium rises during the fermentation, and hence enzymes required for the fermentation may be inactivated or the productive bacteria may become extinct. Therefore, it is necessary to cool the medium during the fermentation.

On the other hand, enzymes and proteins produced by thermotolerant bacteria are generally stable at elevated temperatures. Therefore, their applications as diagnostic agents, catalysts for industrial use and so forth have been highly developed. Moreover, there has also been expected utilization of the thermotolerant bacteria themselves for the fermentative production. If fermentative production of L-lysine at elevated temperatures can be realized by using such thermotolerant bacteria, the need for cooling of the medium can be eliminated, and thus the cost for the cooling during the fermentation can be reduced. Moreover, it is considered that, if it becomes possible to perform the fermentation at elevated temperatures, the reaction rate can also be improved.

Like the microorganisms usually used for the aforementioned fermentative production of amino acids and so forth, heterotrophic organisms requiring organic compounds as a carbon source often utilize saccharides metabolized by the glycolysis system as the carbon source. On the other hand, alcohols such as ethanol and methanol, cellulose, carbon dioxide and so forth are also researched as the carbon source for the fermentation. These carbon sources are chosen mainly for an economical reason, because of the current high cost ratio of the raw materials in the fermentative production. However, from the viewpoints of technical diversification and development of prospective techniques, it is thought that productions of amino acids by fermentation using these carbon sources shall constitute important techniques.

As a thermotolerant bacterium, an L-lysine-producing bacterium, which is a mutant strain of a vitamin B₁₂ auxotrophic *Bacillus* sp., MGA3 strain, has been reported (WO90/12105; Lee, G.H., et al., Biotechnology and Bioengineering, 49, 639-653 (1996)). However, there has been no report of L-lysine production for a thermotolerant *Bacillus* bacterium which is vitamin B₁₂ non-auxotrophic.

### SUMMARY OF THE INVENTION

The present invention has been accomplished from the viewpoint of the aforementioned technical status, and an object thereof is to provide a method for producing L-lysine by fermentation using a thermotolerant *Bacillus* bacterium which is vitamin B₁₂ non-auxotrophic.

The inventors of the present invention assiduously studied in order to achieve the aforementioned object. As a result, they obtained an L-lysine analogue-resistant mutant strain from a strain of vitamin B₁₂ non-auxotrophic methanol-assimilating thermotolerant bacterium, *Bacillus methanolicus,* and found that the mutant strain has L-lysine-producing ability. Thus, they accomplished the present invention.

That is, the present invention provides the followings.
(1) A method for producing L-lysine, which comprises culturing a methanol-assimilating thermotolerant *Bacillus* bacterium which has L-lysine-producing ability and is vitamin B₁₂ non-auxotrophic in a medium to produce and accumulate L-lysine in a culture and collecting the L-lysine from the culture.
(2) The method according to (1), wherein the bacterium is a bacterium belonging to *Bacillus methanolicus.*
(3) The method according to (2), wherein the bacterium is derived from *Bacillus methanolicus* PB1 (NCIMB13113).
(4) The method according to any one of (1) to (3), wherein the bacterium has resistance to an L-lysine analogue.

In the present invention, the term "L-lysine-producing ability" refers to an ability of a bacterium used for the present invention to produce and accumulate L-lysine in a medium, when the bacterium is cultured in the medium.

According to present invention, L-lysine can be produced by using a thermotolerant *Bacillus* bacterium which is vitamin B₁₂ non-auxotrophic. According to the present invention, L-lysine can be produced by using a carbon source such as methanol without adding vitamin B₁₂ to the medium.

### DETAILED DESCRIPTION OF THE INVENTION

Hereafter, the present invention will be explained in detail.

The bacterium used for the method for producing L-lysine according to the present invention is a bacterium which belongs to the genus *Bacillus,* is thermotolerant, assimilates methanol, has L-lysine-producing ability and is vitamin B₁₂ non-auxotrophic. As an example of such a methanol-assimilating thermotolerant *Bacillus* bacterium which is vitamin B₁₂ non-auxotrophic, *Bacillus methanolicus* can be mentioned. As a specific example of *Bacillus methanolicus, Bacillus methanolicus* PB1 (NCIMB13113) can be mentioned. Anyone can obtain the *Bacillus methanolicus* PB1 strain (Arfman, N. et al, Int. J. Syst. Bacteriol., 42(3), 439 (1992)) from NCIMB (National Collections of Industrial and Marine Bacteria Ltd., Address: 23 St. Machar Drive, Aberdeen AB2 1RY, Scotland, United Kingdom).

The bacterium used for the present invention can be obtained by imparting the L-lysine-producing ability to such a methanol-assimilating thermotolerant *Bacillus* bacterium which is vitamin B₁₂ non-auxotrophic as mentioned above (henceforth also referred to simply as "thermotolerant *Bacillus* bacterium"). A thermotolerant *Bacillus* bacterium having L-lysine-producing ability can be obtained by isolating an L-lysine analogue resistant mutant strain from wild strains of thermotolerant *Bacillus* bacteria. As the L-lysine analogue, there can be mentioned S-2-aminoethylcysteine (AEC), γ-methyllysine (ML), α-amino-β-hydroxyvaleric acid (AHV) and trans-4,5-dehydrolysine (DHL). A method for obtaining a thermotolerant *Bacillus* bacterium having L-lysine analogue resistance will be explained below.

A thermotolerant *Bacillus* bacterium having L-lysine analogue resistance can be obtained by culturing thermotolerant *Bacillus* bacteria in a minimum medium containing an L-lysine analogue at a concentration which normally inhibits their growth, and selecting a strain grown in the medium. The term "inhibition of growth" includes retardation and prevention of growth. The selection may be performed once or two or more times. While the amount of the L-lysine analogue added to the medium is not particularly limited so long as it gives a concentration which inhibits the growth of a parent strain to which L-lysine analogue resistance is to be imparted, for example, it may be 0.01 mg/ml or more, preferably 1 mg/ml to 50 mg/ml. Prior to the selection, the thermotolerant *Bacillus* bacteria may be subjected to a mutagenesis treatment. The mutagenesis treatment can be performed by using UV irradiation, or a mutagenizing agent usually used for artificial mutagenesis such as N-methyl-N'-nitro-N-nitrosoguanidine (NTG) and nitrous acid. The selection of an L-lysine analogue resistant strain may be performed for one L-lysine analogue or two or more L-lysine analogues. Moreover, the selection for one L-lysine analogue may be performed once or two or more times.

A thermotolerant *Bacillus* bacterium having L-lysine analogue resistance obtained as described above can grow even in the presence of L-lysine analogue at a concentration at which the parent strain cannot grow.

The L-lysine-producing ability can also be imparted to a thermotolerant *Bacillus* bacteria by using a mutation other than the L-lysine analogue resistance mutation, as known for L-lysine-producing bacteria of *Brevibacterium* or *Corynebacterium* bacteria. As mutant strains containing such a mutation, there have been known, for example, mutant strains exhibiting auxotrophy for amino acids such as L-leucine, L-homoserine, L-proline, L-serine, L-arginine, L-alanine and L-valine (U.S. Patent Nos. 3,708,395 and 3,825,472), L-lysine-producing mutants that show resistance to DL-α-amino-ε-caprolactam, α-amino-lauryllactam, aspartic acid analogue, sulfa drug, quinoid and N-lauroylleucine, L-lysine-producing mutant strains that show resistance to an inhibitor of oxaloacetate decarboxylase or a respiratory enzyme (Japanese Patent Laid-open (Kokai) Nos. 50-53588, 50-31093, 52-102498, 53-9394, 53-86089, 55-9783, 55-9759, 56-32995, 56-39778, Japanese Patent Publication (Kokoku) Nos. 53-43591 and 53-1833), L-lysine-producing mutant strains which exhibiting auxotrophy for inositol or acetate (Japanese Patent Laid-open Nos. 55-9784 and 56-8692), L-lysine-producing mutants that are susceptible to fluoropyruvic acid (Japanese Patent Laid-open Nos. 55-9783 and 53-86090), mutants that show resistance to ethylene glycol (U.S. Patent No. 4,411,997) and so forth.

Further, the L-lysine-producing ability can also be imparted or enhanced by enhancing an L-lysine biosynthesis enzyme gene of the thermotolerant *Bacillus* bacterium. As the L-lysine biosynthesis enzyme gene, there can be mentioned an aspartokinase gene, a dihydrodipicolinate reductase gene, a dihydrodipicolinate synthase gene, a diaminopimelate decarboxylase gene, a diaminopimelate dehydrogenase gene, a phosphoenolpyruvate carboxylase gene and so forth.

The aforementioned enzyme genes can be obtained from, for example, a gene library of thermotolerant *Bacillus* bacteria as a gene which restores auxotrophy of a mutant strain of microorganism deficient in an enzyme in question. There are disclosed genes obtained as described above, i.e., the gene coding for aspartokinase II (U.S. Patent No. 5,243,039) and the gene coding for diaminopimelate decarboxylase (U.S. Patent No. 5,426,052) derived from a vitamin B₁₂ auxotrophic thermotolerant *Bacillus* bacterium, *Bacillus* sp. MGA3 strain. In the present invention, while these genes derived from a thermotolerant *Bacillus* bacterium which is vitamin B₁₂ auxotrophic can be used, genes similarly obtained from a thermotolerant *Bacillus* bacterium which is vitamin B₁₂ non-auxotrophic can also be used similarly.

As a vector for introducing an L-lysine biosynthesis gene into a thermotolerant *Bacillus* bacterium, there can be mentioned pUB110, pHY300PLK, pHV1248, pE194, pC194, pBC16, pSA0501, pSA2100, pAM77, pT181, pBD6, pBD8, pBD64, pHV14 and their derivatives and so forth. Moreover, an endogenous plasmid harbored by the *Bacillus methanolicus* PB1 strain, which has a size of about 20.5 kbp, and its derivatives can also be used for introducing an L-lysine biosynthesis gene into a thermotolerant *Bacillus* bacterium (Japanese Patent Application No. 11-105812/1999).

Transformation of thermotolerant *Bacillus* bacteria can be attained by the methods comprising introduction of a recombinant DNA into host cells as DNA recipient bacteria, which are made into protoplasts or spheroplasts (Chang, S. and Choen S.N., Molec. Gen., Genet., 168, 111 (1979); Bibb, M.J., Ward, J.M. and Hopwood, O.A., Nature, 274, 398 (1978); Hinnen, A., Hicks, J.B. and Fink G.R., Proc. Natl. Acad. Sci. USA, 75, 1929 (1978)).

L-Lysine can be produced by culturing a methanol-assimilating thermotolerant *Bacillus* bacterium which is vitamin B₁₂ auxotrophic, obtained as described above in a medium to produce and accumulate L-lysine in a culture, and collecting the L-lysine from the culture.

The medium used for the culture may be a usual medium containing a carbon source, a nitrogen source, inorganic ions, and other organic components if needed.

As the carbon source, there can be used saccharides such as glucose, maltose, and ribose, alcohols such as methanol, mannitol and sorbitol and so forth. In the present invention, methanol can particularly suitably be used.

As the nitrogen source, inorganic ammonium salts such as ammonium sulfate, ammonium chloride and ammonium phosphate, organic nitrogen such as soybean hydrolysate, ammonia gas, aqueous ammonia and so forth can be used.

As the inorganic ions or sources thereof, a small amount of potassium phosphate, magnesium sulfate, iron ions, manganese ions and so forth may be added. As a trace amount organic nutrient, it is desirable to add a suitable amount of required substances such as biotin, folic acid and vitamin B₁, yeast extract and so forth as required.

The culture is performed under conditions suitable for growth of a microorganism to be used. In general, it is preferably performed for 16 to 72 hours under an aerobic condition, and the culture temperature is controlled to be 20 to 45°C, and pH to be 5 to 8.5 during the culture. For adjusting pH, inorganic or organic acidic or alkaline substances, ammonia gas and so forth can be used. Further, when a thermotolerant bacterium is used as a host, it can be cultured at a culture temperature of 42 to 60°C.

Collection of L-lysine from the culture can usually be carried out by using a combination of known techniques such as techniques using ion exchange resins, precipitation methods and so forth.

### EXAMPLES

Hereafter, the present invention will be further specifically explained with reference to the following examples.

### Example 1: Acquisition of L-lysine analogue resistant strain from Bacillus methanolicus PB1 strain

A vitamin B₁₂ non-auxotrophic methanol assimilating thermotolerant bacterium, *Bacillus methanolicus* PB1 (NCIMB13113), was inoculated into a test tube containing 5 ml of Medium A (K₂HPO₄ 3.8 g/L, NaH₂PO₄·2H₂O 3.1 g/L, (NH₄)₂SO₄ 3.6 g/L, MgSO₄·7H₂O 0.5 g/L, FeSO₄·7H₂O 2 mg/L, CuSO₄·5H₂O 40 µg/L, H₃BO₃ 30 µg/L, MnSO₄·4H₂O 200 µg/L, ZnSO₄·7H₂O 200 µg/L, Na₂MoO₄·2H₂O 40 µg/L, CaCl₂·2H₂O 5.3 µg/L, CoCl₂·6H₂O 40 µg/L, thiamine hydrochloride 100 µg/L, calcium pantothenate 100 µg/L, riboflavin 100 µg/L, biotin 100 µg/L, nicotinic acid 100 µg/L, pyridoxine hydrochloride 100 µg/L, aminobenzoic acid 20 µg/L, lipoic acid 20 µg/L, folic acid 20 µg/L and methanol 2% (V/V)), and cultured overnight at 50°C with shaking.

The above culture broth was inoculated into 50 ml of Medium A contained in a Sakaguchi flask at a concentration of 1%, and allowed to grow to a logarithmic growth phase at 50°C, and then cells were harvested.

The cells obtained as described above were washed with potassium phosphate buffer (pH 7.4), then suspended in potassium phosphate buffer (pH 7.4) containing 50 mg/L N-methyl-N'-nitro-N-nitrosoguanidine (NTG), and left for 30 minutes at 50°C. The cells subjected to the NTG treatment were washed twice with potassium phosphate buffer (pH 7.4), then suspended in Medium A, and cultured overnight with shaking. A portion of the culture broth was collected, applied to Agar Medium A (Medium A containing 1.5% of agar) containing 1 mg/ml of S-2-aminoethylcysteine (AEC), and cultured at 50°C for 2 to 5 days. The emerged colonies were streaked and cultured at 50°C on Agar Medium A containing AEC at the same concentration as above. Then, each formed colony was inoculated into a test tube containing 5 ml of Medium A, and cultured for 2 to 5 days with shaking. When each culture broth was analyzed by thin layer chromatography (TLC) using a developing solution that had a composition of n-butanol:acetic acid:water:diethylamine = 5:5:2:1 (by volume), a spot of L-lysine could be detected for some samples. Two strains for which large spots of L-lysine were observed were selected, and designated as #1691 and #1821, respectively.

#1691 and #1821 were designated as AJ13602 and AJ13603, respectively. They were deposited at the National Institute of Bioscience and Human-Technology, Agency of Industrial Science and Technology, Ministry of International Trade and Industry (postal code: 305-8566, 1-3 Higashi 1-Chome, Tsukuba-shi, Ibaraki-ken, Japan) on June 4, 1999, and received the accession numbers of FERM P-17413 and FERM P-17414, respectively. Then, they were transferred to international depositions under the provisions of the Budapest Treaty on June 2, 2000 and received accession numbers of FERM BP-7182 and FERM BP-7183, respectively.

### Example 2: Production of L-lysine with L-lysine analogue resistant strain

The aforementioned two strains obtained in Example 1 (#1691, #1821) were cultured at 50°C for two days on Agar Medium A, and one platinum loop of the cells of each stain were inoculated into a test tube containing 5 ml of Medium A, and cultured at 50°C for two days with shaking.

Then, 2 ml of each culture broth was added to a Sakaguchi flask containing 20 ml of Medium A, and cultured similarly at 50°C for 2 to 3 days with shaking. After the culture was completed, the cells were removed by centrifugation, and the concentration of L-lysine contained in the culture supernatant was determined by an amino acid analyzer. The results are shown in Table 1.

**Table 1**

| Strain | L-Lysine production amount (g/L) |
|---|---|
| PB1 | 0 |
| #1691 | 0.36 |
| #1821 | 0.64 |

Further, the strains were similarly cultured by using a jar fermenter of 1-L volume. The medium used contained Medium A as a base medium. A portion of the medium was periodically withdrawn during the culture, and its methanol concentration was analyzed by gas chromatography. When the methanol concentration became 0.5 to 1.0% (V/V), methanol was appropriately added to the medium.

The pH of the medium was maintained at 6.7 by addition of ammonia, and foaming was controlled by appropriately adding a silicone anti-foaming agent (GD-113). After the culture was completed, the concentration of L-lysine in the medium was determined in the same manner as above. The culture times and concentrations of L-lysine HCl accumulated in the medium are shown in Table 2. In the table, "-" means "not tested".

**Table 2**

| Strain | Production amount of L-lysine HCl (g/L) | | | |
|---|---|---|---|---|
| | 14 hours | 27 hours | 50 hours | 56 hours |
| #1691 | 0.34 | 1.8 | - | 3.8 |
| #1821 | 0.36 | 2.1 | 4.5 | - |

## Claims

1. A method for producing L-lysine, which comprises culturing a methanol-assimilating thermotolerant Bacillus bacterium which has L-lysine-producing ability and is vitamin B₁₂ non-auxotrophic.

2. The method according to claim 1, wherein the bacterium is a bacterium belonging to Bacillus methanolicus.

3. The method according to claim 2, wherein the bacterium is derived from Bacillus methanolicus PB1 (NCIMB13113).

4. The method according to any one of the claims 1 to 3, wherein the bacterium has resistance to an L-lysine analogue.

5. The method according to any one of the claims 1 to 4, wherein the Bacillus bacterium is a bacterium having the accession number FERM BP-7182 or FERM BP-7183.

6. A methanol-assimilating thermotolerant Bacillus bacterium which has L-lysine-producing ability and is vitamin B₁₂ non-auxotrophic.

7. The bacterium according to claim 6, which is a bacterium belonging to Bacillus methanolicus.

8. The bacterium according to claim 7, which has the accession number FERM BP-7182 or FERM BP-7183.
